# EUROPEAN PATENT APPLICATION

(11) **EP 4 541 410 A2**
(43) Date of publication of application: **23.04.2025**
(21) Application number: 25154252.8
(22) Date of filing: 21.12.2020
(51) Int. Cl.: A61M 60/216

(54) **BLOOD PUMP DISTAL OUTFLOW CAGE**

(30) Priority: 31.12.2019 US 201962955603 P
(62) Divisional of application: 20842845.8
(71) Applicant: Abiomed, Inc., Danvers, MA 01923 (US)
(72) Inventor: BERNAZANI, Michael J., Danvers (US)
(74) Representative: Klunker IP Patentanwälte PartG mbB

(57) **Abstract**

Blood pump assemblies with blood outflow cage components designed to reduce stress on a patient's vasculature, reduce hemolysis and damage to blood, and improve manufacturability. The blood pump assembly includes a blood outflow cage designed to expel the blood from a distal end of a blood pump assembly. The openings in the blood outflow cage have edges, corners, and junctions that are rounded, and smoothly transition into a diffuser at the proximal end of the blood outflow cage. The struts of the blood outflow cage may further taper down in thickness as they transition into a thinner cylindrical section at the proximal end of the blood outflow cage.

## Description

### CROSS REFERENCE TO RELATED APPLICATION

The present application claims priority to U.S. Provisional Application No. 62/955,603 filed December 31, 2019, the disclosure of which is incorporated by reference in its entirety.

### TECHNICAL FIELD

The present disclosure relates to blood pump assemblies. More specifically, the present disclosure relates to an outflow cage component arranged at the distal end of a blood pump assembly.

### BACKGROUND

A blood pump assembly, such as an intracardiac blood pump assembly, is introduced into the heart to deliver blood from the heart into an artery. For example, when used for right heart support, blood pump assemblies can pull blood from the inferior vena cava and expel blood into the pulmonary artery. In some cases, the blood pump assemblies can be inserted via a catheterization procedure through the femoral vein, into the inferior vena cava, through the right atrium, across the tricuspid valve, into the right ventricle, through the pulmonary valve, and into the pulmonary artery. This insertion path is tortuous, requiring the blood pump assembly to be passed through several bends, which can introduce stresses on the patient's vasculature, particularly through contact with the distal end of the blood pump assembly. In addition, the pumping of blood via a blood pump assembly can damage the blood or cause hemolysis as the blood is drawn through the blood pump assembly. Finally, as the blood outflow cages described herein incorporate a diffuser at their distal end, the junctions between the struts of the blood outflow cage and the diffuser can further interfere with blood flow, and also complicate manufacturing.

### BRIEF SUMMARY

Devices and methods of manufacture and implementation described herein provide blood pump assemblies with outflow cage components designed to reduce stress on the patient's vasculature, reduce hemolysis and damage to blood, and improve manufacturability. The blood pump assembly includes a blood outflow cage designed to expel the blood from a distal end of a blood pump assembly. The blood outflow cage has edges, corners, and junctions that are rounded. This provides several benefits. Rounding the outer edges of the blood outflow cage reduces stress and abrasion on vasculature as the blood pump assembly is being inserted into the patient and advanced to its operative location. Rounding the inner and outer edges of the blood outflow cage also leads to a significant reduction in hemolysis as described in U.S. Patent No. 9,433,713, which is incorporated by reference herein. In addition, as the blood outflow cages described herein incorporate a diffuser at their distal end, the use of fillets on the junctions between the struts and the diffuser (i.e. the corners at the intersection of the struts and the diffuser) further improves blood flow around those junctions. This also causes fewer red blood cells to be ruptured as the blood flows through the blood outflow cage, resulting in further reductions in hemolysis. Finally, the rounded edges, corners, and junctions of the blood outflow cages described herein improve manufacturability of the blood outflow cage. In particular, these features are conducive to manufacturing using automated machining processes, and allow for the entire blood outflow cage to be formed from a single workpiece.

In one aspect, the disclosure describes a blood outflow cage for an intravascular blood pump assembly and sized for passage through a vascular lumen of a patient. The assembly includes a distal cap with a substantially conical, substantially curved-conical, or substantially dome-shaped outer surface; a diffuser having a substantially conical or substantially curved-conical outer surface. The diffuser is disposed proximal to the distal cap. The assembly has a peripheral wall having an inner surface; an outer surface; two or more blood exhaust openings, each blood exhaust opening being defined by two struts, wherein each strut has rounded inner and outer edges and rounded junctions with the diffuser. The proximal end of each blood exhaust opening has rounded corners and a first cylindrical section that abuts the proximal end of each strut. The blood outflow cage optionally has an aperture through the diffuser and the distal cap. In some aspects, the peripheral wall tapers in thickness from a first thickness at the struts to a second thickness at the first cylindrical section, wherein the first thickness is greater than the second thickness. In some aspects, the peripheral wall also has a second cylindrical section that abuts the first cylindrical section, and wherein an outer diameter of the second cylindrical section is smaller than an outer diameter of the first cylindrical section. The blood outflow cage optionally has a flexible tubular extension coupled to the distal cap. In some aspects, the distal end of the flexible tubular extension is curved in its relaxed state. The blood outflow cage optionally has a fillet between an inner surface of each strut and the diffuser. In some aspects, the fillet has a radius of approximately 0.30 mm. In some aspects, the inner edges of the struts of the blood outflow cage each have a first radius between 0.07 mm and 0.21 mm, and the outer edges of the struts of the blood outflow cage each have a second radius between 0.07 mm and 0.21 mm. In some aspects, the blood outflow cage is manufactured from a single piece of metal or polymer.

In another aspect, the disclosure describes a blood pump assembly sized for passage through a vascular lumen of a patient. The blood pump assembly includes: a pump; an impeller blade rotatably coupled to the pump; a blood inflow cage extending about a rotation axis of the impeller blade, and having at least two blood inlet openings; a cannula in fluid communication with the first housing, the proximal end of the cannula being coupled to the blood inflow cage; and a blood outflow cage coupled to the distal end of the cannula. The blood outflow cage includes a distal cap with a substantially conical, substantially curved-conical, or substantially dome-shaped outer surface; a diffuser having a substantially conical or substantially curved-conical outer surface, wherein the diffuser is disposed proximal to the distal cap; and a peripheral wall having: an inner surface; an outer surface; two or more blood exhaust openings, each blood exhaust opening being defined by two struts, wherein each strut has rounded inner and outer edges and rounded junctions with the diffuser, and wherein the proximal end of each blood exhaust opening has rounded corners; and a first cylindrical section that abuts the proximal end of each strut. The blood pump assembly optionally includes an aperture through the diffuser and the distal cap of the blood outflow cage. In some aspects, the peripheral wall of the blood outflow cage tapers in thickness from a first thickness at the struts to a second thickness at the first cylindrical section, wherein the first thickness is greater than the second thickness. In some aspects, the peripheral wall of the blood outflow cage has a second cylindrical section that abuts the first cylindrical section, wherein an outer diameter of the second cylindrical section is smaller than an outer diameter of the first cylindrical section. The blood pump assembly optionally includes a flexible tubular extension coupled to the distal cap of the blood outflow cage. In some aspects, the distal end of the flexible tubular extension is curved in its relaxed state. The blood pump assembly may further include a fillet between an inner surface of each strut and the diffuser of the blood outflow cage. In some aspects, the fillet has a radius of approximately 0.30 mm. In some aspects, the inner edges of the struts of the blood outflow cage each have a first radius between 0.07 mm and 0.21 mm, and the outer edges of the struts of the blood outflow cage each have a second radius between 0.07 mm and 0.21 mm. In some aspects, the blood outflow cage is manufactured from a single piece of metal or polymer.

### BRIEF DESCRIPTION OF THE DRAWINGS

Fig. 1 shows a blood pump assembly in accordance with aspects of the disclosure.
Fig. 2 shows a perspective view of a blood outflow cage in accordance with aspects of the disclosure.
Fig. 3 shows a perspective view of a blood outflow cage in accordance with aspects of the disclosure.
Fig. 4 shows a cross-sectional view of the blood outflow cage in accordance with aspects of the disclosure.
Figs. 5A and 5B show isometric views of the blood outflow cage in accordance with aspects of the disclosure.
Fig. 6 shows a cross-sectional view of the blood outflow cage in accordance with aspects of the disclosure.

### DETAILED DESCRIPTION

The present technology will now be described with respect to the following exemplary systems and methods.

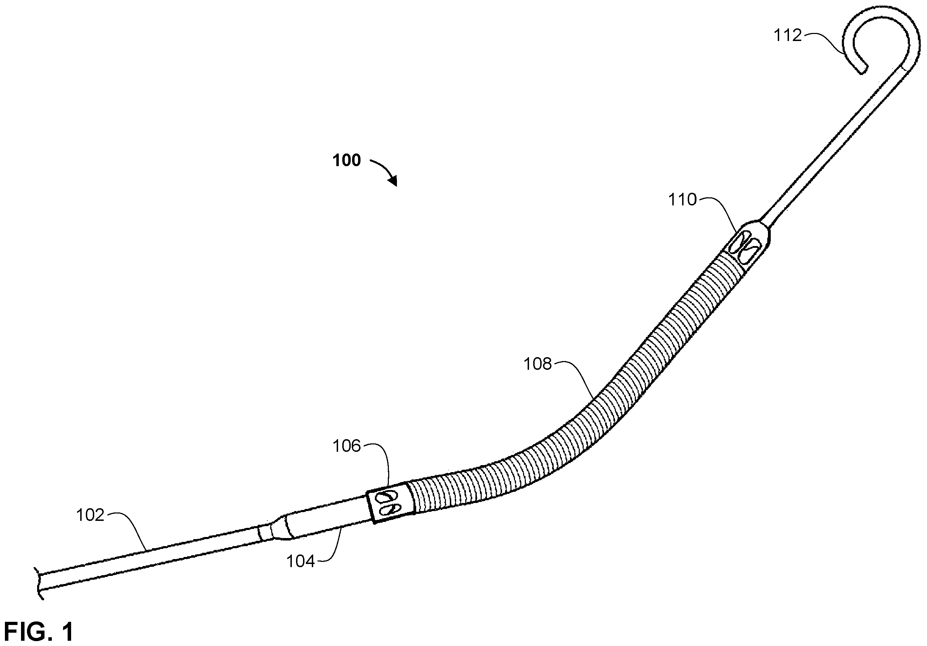

FIG. 1 depicts an exemplary blood pump assembly 100 adapted for right heart support. The blood pump assembly 100 includes a catheter 102, blood pump 104, a blood inflow cage 106, a cannula 108, a blood outflow cage 110, and a pigtail extension 112. The blood pump 104 is coupled to the proximal end of cannula 108 via the blood inflow cage 106. In some cases, the blood inflow cage 106 may encapsulate some or all of blood pump 104, and/or may be integrated into the housing of blood pump 104. The distal end of cannula 108 is further coupled to the blood outflow cage 110. Pigtail extension 112 is coupled to the distal end of the blood outflow cage 110. A catheter 102 is also coupled to the proximal end of blood pump 104.

Blood pump 104 includes a rotatable impeller blade (not shown), which may be driven by an internal motor. For example, blood pump 104 may include an internal electrical micro-axial pump having a pumping capability greater than 4 L/min, and a diameter of 21 or 22 Fr. Blood pump 104 may also be driven by a remote source, such as a motor located outside the patient which is coupled to a flexible drive shaft running through catheter 102.

Catheter 102 may house electrical lines coupling the motor of blood pump 104 to one or more electrical controllers or other sensors. Catheter 102 may also house other components, such as a purge fluid conduit, and/or other conduits configured to receive a guidewire.

The blood inflow cage 106 includes one or more apertures or openings configured to allow blood to be drawn into cannula 108 when the blood pump 104 is operating.

Cannula 108 may include an elongated flexible hose portion. For example, cannula 108 may be composed, at least in part, of a polyurethane material. Cannula 108 may further include a shape-memory coil, such as a nitinol coil. Cannula 108 may be formed such that it includes one or more bends or curves in its relaxed state, or it may be configured to be straight in its relaxed state. Cannula 108 can be any suitable diameter, but will generally be similar in diameter to blood pump 104.

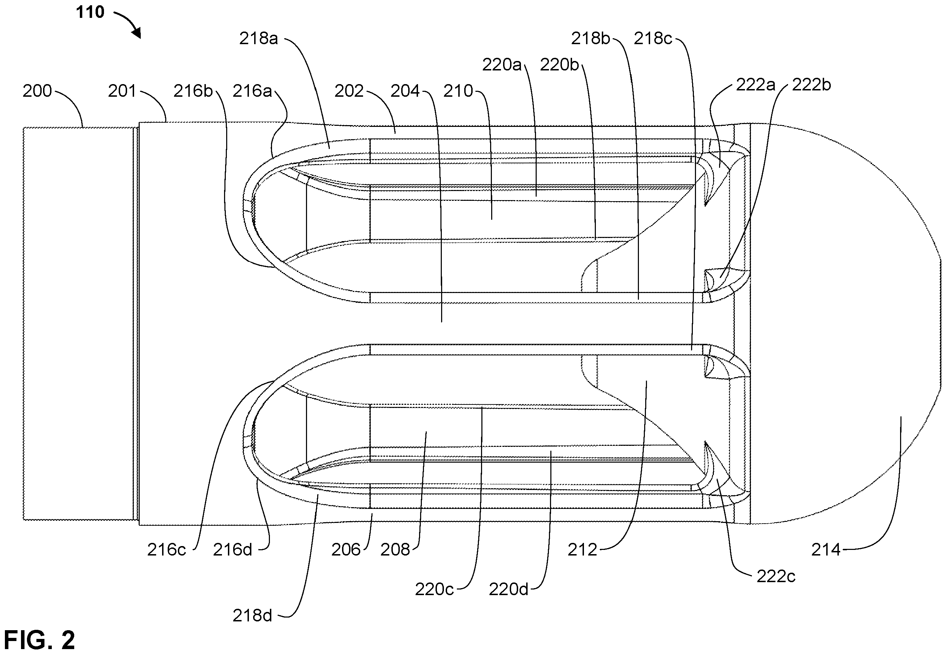

Blood outflow cage 110 includes one or more apertures or openings configured to allow blood to flow from the cannula 108 out of the blood pump assembly 100. Blood outflow cage 110 can be composed of any suitable bio-compatible material. For example, blood outflow cage 110 may be formed out of bio-compatible metals such as stainless steel, titanium, or bio-compatible polymers such as polyurethane. In addition, the surfaces of blood outflow cage 110 may be treated in various ways, including, but not limited to etching, texturing, or coating or plating with another material. For example, the surfaces of blood outflow cage 110 may be laser textured. Although blood outflow cage 110 may be manufactured from multiple pieces, the designs described in more detail below are conducive to manufacturing the blood outflow cage from a single piece of metal or polymer, e.g., by using automated machining processes. Blood outflow cage 110 may also be manufactured using rapid-prototyping or injection-molding processes. Further features of blood outflow cage 110 will be described in detail below with respect to the examples of FIGS. 2-6.

Pigtail extension 112 assists with stabilizing and positioning the blood pump assembly 100 in the correct position in the pulmonary artery. Pigtail extension 112 may be solid or tubular. If tubular, pigtail extension 112 may be configured to allow a guide wire to be passed through it to further assist in the positioning of the blood pump assembly 100. Pigtail extension 112 may be any suitable size. For example, pigtail extension may have an outer diameter in the range of 4-8 Fr. Pigtail extension 112 may be composed, at least in part, of a flexible material, and may be configurable from a straight configuration to a partially curved configuration. Pigtail extension 112 may also have sections with different stiffnesses. For example, pigtail extension 112 may include a proximal section that is stiff enough to prevent it from buckling, thereby keeping the blood outflow cage 110 in the desired location, and a distal section that is softer and has a lower stiffness, thereby providing an atraumatic tip for contact with the pulmonary artery wall and to allow for guidewire loading. In such a case, the proximal and distal sections of the pigtail extension 112 may be composed of different materials, or may be composed of the same material, treated to provide different stiffnesses.

However, pigtail extension 112 is an optional structure. The present technology may also be used with blood pump assemblies that include extensions of different types, shapes, materials, and qualities. Likewise, the present technology may be used with blood pump assemblies that have no extensions of any kind beyond the distal end of the blood outflow cage 110.

Blood pump assembly 100 may be inserted percutaneously. For example, when used for right heart support, blood pump assembly 100 may be inserted via a catheterization procedure through the femoral vein, into the inferior vena cava, through the right atrium, across the tricuspid valve, into the right ventricle, through the pulmonary valve, and into the pulmonary artery. Once positioned in this way, the blood pump assembly 100 delivers blood from the blood inflow cage 106, which sits inside the inferior vena cava, through cannula 108, to the blood outflow cage 110, which sits inside the pulmonary artery.

FIGS. 2-6 depict an exemplary blood outflow cage 110 in accordance with aspects of the technology. All reference numbers shared between FIGS. 2, 3, 4, 5A, 5B, and 6 are meant to denote the same features.

FIG. 2 depicts a perspective view of blood outflow cage 110. The distal end of blood outflow cage 110 includes a diffuser 212 which transitions into a domed end cap 214. At its proximal end, blood outflow cage 110 is comprised of a peripheral wall having an inner and outer surface, a cylindrical section 200, another cylindrical section 201, and five struts 202, 204, 206, 208, and 210. Cylindrical section 200 is adapted to be coupled to cannula 108, and cylindrical section 201 is of slightly larger diameter than cylindrical section 200. The distal end of cylindrical section 201 transitions into five struts 202, 204, 206, 208, and 210, which define five openings through which blood may exit blood outflow cage 110. While the exemplary blood outflow cage 110 of FIG. 2 is shown with five struts, any number of two or more struts may be used. Struts 202, 204, 206, 208, and 210 connect to the distal end of the diffuser 212.

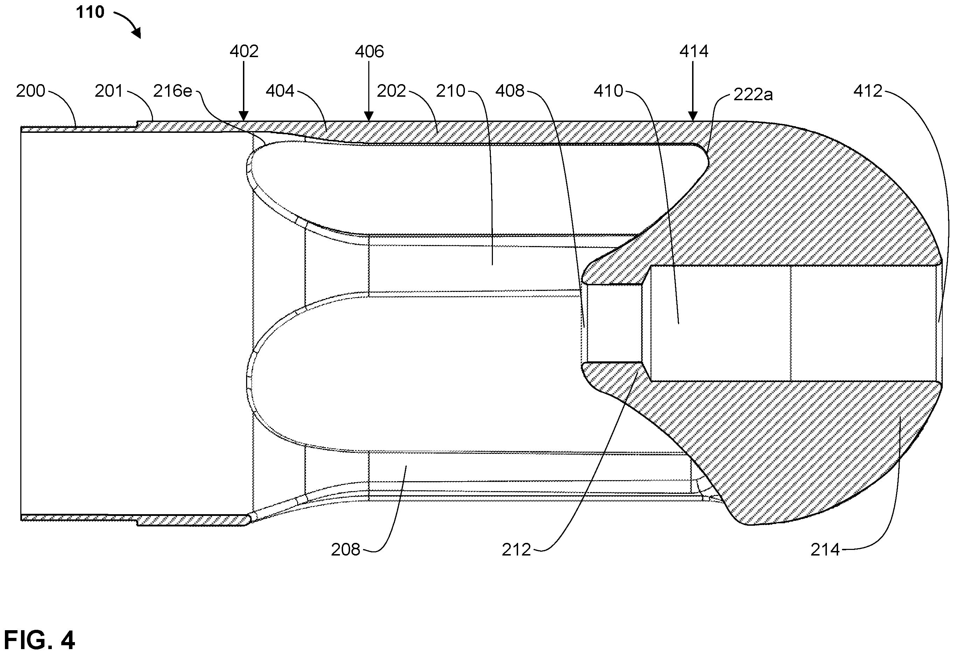

Diffuser 212 is adapted to direct blood flow out of blood outflow cage 110. Thus, while diffuser 212 has a curved-conical shape in the examples of FIGS. 2-6, other shapes may be employed, such as a conical shape with no curvature. Likewise, while diffuser 212 is shown in FIGS. 2-6 as having a smooth surface, it may include vanes, fins, or other features adapted to further aid blood flow out of the blood outflow cage 110. Finally, while diffuser 212 is shown in FIGS. 2-6 as having a blunted proximal tip, it may alternatively come to a pointed tip in alternatives where there is no aperture (such as aperture 410 of FIG. 4) running through the distal end of blood outflow cage 110.

End cap 214 is depicted as having a dome-shape. However, end cap 214 may have any shape conducive to inserting blood pump assembly 100 into a vascular lumen of a patient, such as a conical or curved-conical shape, or a cylindrical profile with rounded edges.

The inner and outer edges of struts 202, 204, 206, 208, and 210 are rounded. For example, in FIG. 2, rounded outer edges 218a, 218b, 218c, and 218d can be seen on struts 202, 204, and 206, respectively. Likewise, rounded inner edges 220a, 220b, 220c, and 220d can be seen on struts 210 and 208, respectively. Rounding the outer edges of the blood outflow cage 110 reduces stress and abrasion on vasculature as the blood pump assembly is being inserted. This is especially beneficial in implementations in which the blood pump assembly 100 is inserted percutaneously and used for right heart support, which requires the blood pump assembly 100 to be advanced through a tortuous pathway as it passes through the heart to its operational location. In addition, rounding the inner and outer edges of the blood outflow cage 110 also leads to a significant reduction in hemolysis, as described in U.S. Patent No. 9,433,713. The outer edges (e.g., 218a, 218b, 218c, and 218d) and inner edges (e.g., 220a, 220b, 220c, and 220d) are filleted and may have any suitable radius. For example, but without limitation, for a blood outflow cage 110 with an outer diameter of 21 Fr (7 mm), the edges of each strut may be rounded to radii between 0.07 mm and 0.21 mm. In that regard, the inner edges and outer edges need not have the same radii. For example, the inner edges of each strut may be rounded to a radius of approximately 0.08 mm, while the outer edges may be rounded to a radius of approximately 0.20 mm.

The shape of the openings defined by struts 202, 204, 206, 208, and 210 are also rounded. For example, in the opening defined by struts 202 and 204, the proximal end has rounded corners 216a and 216b, and the distal end has rounded junctions 222a and 222b where the struts transition into diffuser 212. Likewise, in the opening defined by struts 204 and 206, the proximal end has rounded corners 216c and 216d, and the distal end has rounded junctions 222b and 222c where the struts transition into diffuser 212. Rounded junctions 222a, 222b, and 222c fan out both tangentially and radially, such that all surfaces and edges of the struts have a smooth transition into diffuser 212. Rounding the shape of the openings of the blood outflow cage 110 in this way further improves blood flow. In particular, using filleted junctions between the struts and the conical surface of diffuser 212 avoids squared corners and pockets that can impart shear stresses on the blood cells and cause hemolysis. The rounded corners of the openings may have any suitable shape and dimension. For example, but without limitation, for a blood outflow cage 110 with an outer diameter of approximately 21 Fr (7 mm), the proximal corners (e.g., 216a, 216b, 216c, and 216d) may have an asymmetric radius in which a major radius of approximately 2 mm transitions into a minor radius of approximately 1.431, with a conic Rho of approximately 0.35. In addition, the rounded junctions (e.g., 222a, 222b, and 222c) may have a radius of approximately 0.3 mm. The use of asymmetric radii can create compound shapes that provide a smooth transition surface for blood flow as it exits the window. Nevertheless, the windows can also be formed using one or more constant radii.

In addition to the benefits described above, rounding the edges, corners, and junctions of the blood outflow cages described herein leads to improvements in manufacturability. In particular, as automated machining processes rely upon rotating milling bits and cutting tools, these features are conducive to manufacturing using automated machining processes. In addition, by using filleted transitions at the junctions between the struts 202, 204, 206, 208, 210 and the diffuser 212, the junctions are made more robust so that automated machining is more feasible, and blood outflow cage 110 can be machined from a single workpiece without the need for welds or other joining methods.

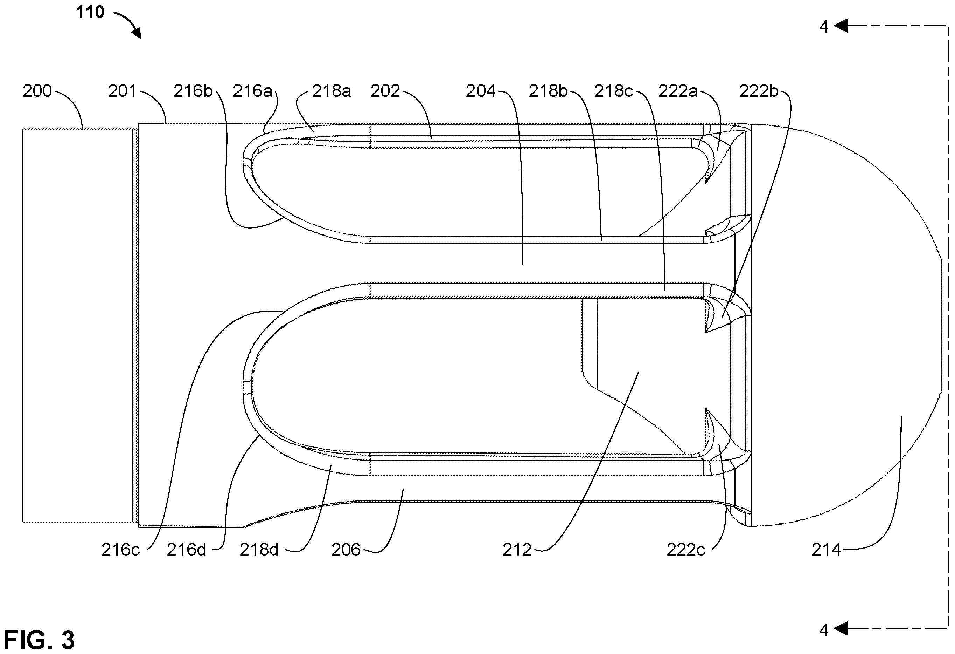

FIG. 3 depicts a second perspective view of the blood outflow cage 110 depicted in FIG. 2. Specifically, FIG. 3 depicts the exemplary blood outflow cage 110 of FIG. 2, rotated so that strut 202 is at the top of blood outflow cage 110. As a result of this orientation, only struts 202, 204, and 206 are visible. In this view, struts 208 and 210 are hidden behind struts 206 and 204, respectively.

FIG. 4 depicts a cross-sectional view of the blood outflow cage 110, oriented as depicted in FIG. 3, and sectioned down its middle along the plane indicated by reference line 4-4 of FIG. 3. By virtue of this sectioning, struts 204 and 206 are no longer visible, and struts 208 and 210 are now visible. In addition, in this view, an aperture 410 is visible running through the distal end of blood outflow cage 110. In particular, the aperture 410 runs from a proximal opening 408 in diffuser 212 to a distal opening 412 in end cap 214. Aperture 410 is adapted to allow a guide wire to be passed through the distal end of blood outflow cage 110. In addition, a distal section of aperture 410 has a larger diameter adapted to couple with a tubular pigtail extension 112. However, like pigtail extension 112, aperture 410 is an optional feature, and the present technology may be used with blood outflow cages that do not include aperture 410, or which include apertures of different configurations.

The sectioned surfaces of blood outflow cage 110 are shown in FIG. 4 with diagonal hatching. This reveals another feature of struts 202, 204, 206, 208, and 210. In particular, as shown with respect to sectioned strut 202, the strut includes a tapered section 404. As can be seen, strut 202 has a substantially constant thickness between arrow 414 and arrow 406, and then has a tapered section 404 as the strut 202 transitions into the thinner wall thickness of cylindrical section 201 at arrow 402. This tapered section 404 corresponds to the fanning out of strut 202 at the rounded corners 216e and 216a (not visible in this sectional view) of the adjacent openings. Tapering the wall thickness in conjunction with the fanning out each strut allows the design to minimize proximal wall thicknesses while still adequately transitioning stress from the struts onto the cylindrical section 201. This tapered section 404 may have any suitable contour and dimension. For example, tapered section 404 may taper in a linear or curved manner. If curved, tapered section 404 may be formed using a single radius, or by blending two or more radii. Thus, for a design similar to that shown in FIG. 4, and assuming a blood outflow cage 110 with an outer diameter of approximately 21 Fr (7 mm), tapered section 404 may include (moving distal to proximal) a concave radius of approximately 6.5 mm that transitions into a convex radius of approximately 4.8 mm. Likewise, the wall thickness may taper from approximately 0.4 mm at or about arrow 406 to approximately 0.2 mm at or about arrow 402.

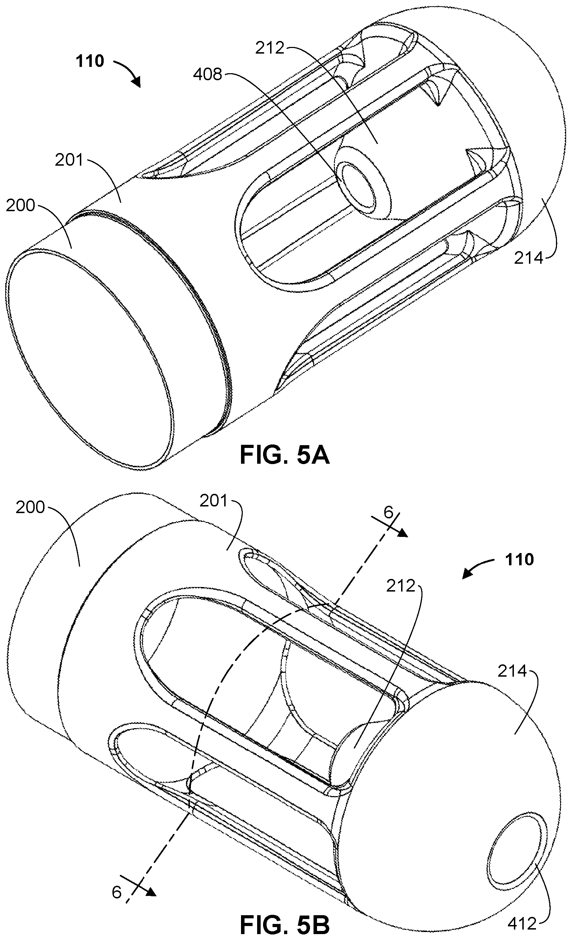

FIGS. 5A and 5B depict isometric views of the blood outflow cage 110 depicted in FIGS. 2-4. Specifically, FIG. 5A depicts an isometric view of the exemplary blood outflow cage 110 of FIGS. 2-4 looking in the distal direction, and FIG. 5B depicts an isometric view of the exemplary blood outflow cage 110 of FIGS. 2-4 looking in the proximal direction.

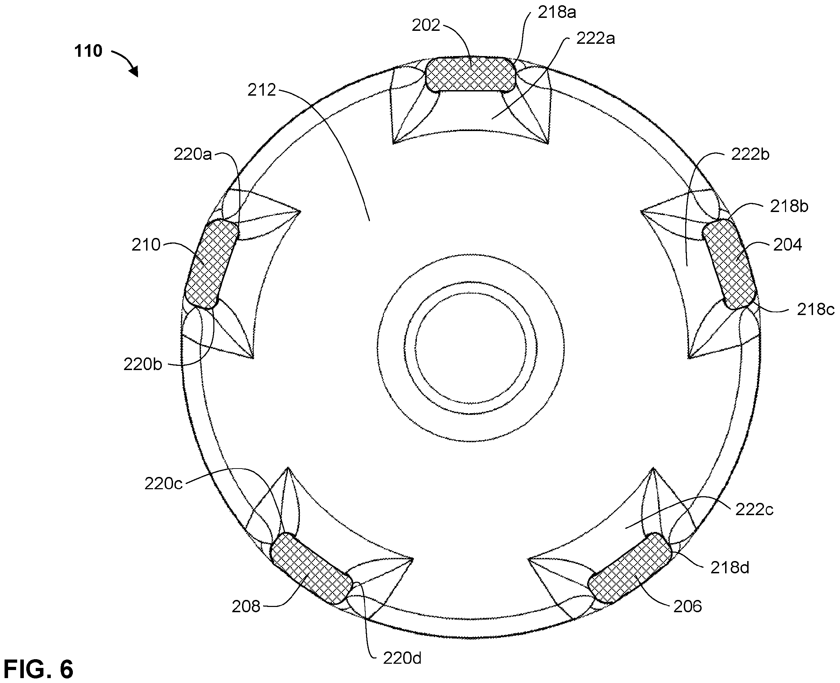

FIG. 6 depicts a cross-sectional view of the blood outflow cage 110, sectioned along line 6-6 of FIG. 5B looking in the direction of the arrows, i.e., towards the distal end of blood outflow cage 110. The sectioned surface of each strut of the blood outflow cage 110 is shown in FIG. 6 with cross hatching.

As utilized herein, the terms "approximately," "about," "substantially" and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described without restricting the scope of these features to the precise numerical ranges provided. Accordingly, these terms should be interpreted as indicating that insubstantial or inconsequential modifications or alterations of the subject matter described and are considered to be within the scope of the disclosure.

For the purpose of this disclosure, the term "coupled" means the joining of two members directly or indirectly to one another. Such joining may be stationary or moveable in nature. Such joining may be achieved with the two members or the two members and any additional intermediate members being integrally formed as a single unitary body with one another or with the two members or the two members and any additional intermediate members being attached to one another. Such joining may be permanent in nature or may be removable or releasable in nature.

It is important to note that the constructions and arrangements of apparatuses or the components thereof as shown in the various exemplary implementations are illustrative only. Although only a few implementations have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter disclosed. For example, elements shown as integrally formed may be constructed of multiple parts or elements, the position of elements may be reversed or otherwise varied, and the nature or number of discrete elements or positions may be altered or varied. The order or sequence of any process or method steps may be varied or re-sequenced according to alternative implementations. Other substitutions, modifications, changes and omissions may also be made in the design, operating conditions and arrangement of the various exemplary implementations without departing from the scope of the present disclosure.

While various implementations have been described and illustrated herein, those of ordinary skill in the art will readily envision a variety of other mechanisms and/or structures for performing the function and/or obtaining the results and/or one or more of the advantages described herein, and each of such variations and/or modifications is deemed to be within the scope of the inventive implementations described herein. More generally, those skilled in the art will readily appreciate that, unless otherwise noted, any parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive implementations described herein. It is, therefore, to be understood that the foregoing implementations are presented by way of example only and that, within the scope of the appended claims and equivalents thereto, inventive implementations may be practiced otherwise than as specifically described and claimed. Inventive implementations of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

The indefinite articles "a" and "an," as used herein in the specification and in the claims, unless clearly indicated to the contrary, should be understood to mean "at least one." As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of" will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of" "only one of" or "exactly one of."

As used herein in the specification and in the claims, the phrase "at least one," in reference to a list of one or more elements, should be understood to mean at least one element selected from any one or more of the elements in the list of elements. The phrase "at least one" should not be understood to require at least one of each and every element specifically listed within the list of elements. The phrase "at least one" should also not be understood to exclude any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one implementation, to at least one, optionally including more than one, A, with no B present (and optionally including elements other than B); in another implementation, to at least one, optionally including more than one, B, with no A present (and optionally including elements other than A); in yet another implementation, to at least one, optionally including more than one, A, and at least one, optionally including more than one, B (and optionally including other elements); etc.

In the claims, as well as in the specification above, all transitional phrases such as "comprising," "including," "carrying," "having," "containing," "involving," "holding," "composed of," "such as," and the like are to be understood to be open-ended, i.e., to mean including but not limited to.

The claims should not be read as limited to the described order or elements unless stated to that effect. It should be understood that various changes in form and detail may be made by one of ordinary skill in the art without departing from the spirit and scope of the appended claims. All implementations that come within the spirit and scope of the following claims and equivalents thereto are claimed.
Preferred embodiments of the invention are described in the following numbered paras 1 to 20:
1. A blood outflow cage for an intravascular blood pump assembly and sized for passage through a vascular lumen of a patient, comprising:
   a distal cap with a substantially conical, substantially curved-conical, or substantially dome-shaped outer surface;
   a diffuser having a substantially conical or substantially curved-conical outer surface, wherein the diffuser is disposed proximal to the distal cap; and
   a peripheral wall having:
      an inner surface;
      an outer surface;
      two or more blood exhaust openings, each blood exhaust opening being defined by two struts, wherein each strut has rounded inner and outer edges and rounded junctions with the diffuser, and wherein the proximal end of each blood exhaust opening has rounded corners; and
      a first cylindrical section that abuts the proximal end of each strut.
2. The blood outflow cage of para 1, further comprising an aperture through the diffuser and the distal cap.
3. The blood outflow cage of para 1, wherein the peripheral wall tapers in thickness from a first thickness at the struts to a second thickness at the first cylindrical section, wherein the first thickness is greater than the second thickness.
4. The blood outflow cage of para 1, wherein the peripheral wall further comprises a second cylindrical section that abuts the first cylindrical section, and wherein an outer diameter of the second cylindrical section is smaller than an outer diameter of the first cylindrical section.
5. The blood outflow cage of para 1, further comprising a flexible tubular extension coupled to the distal cap.
6. The blood outflow cage of para 5, wherein the distal end of the flexible tubular extension is curved in its relaxed state.
7. The blood outflow cage of para 1, further comprising a fillet between an inner surface of each strut and the diffuser.
8. The blood outflow cage of para 7, wherein the fillet has a radius of approximately 0.30 mm.
9. The blood outflow cage of para 1, wherein the inner edges of the struts of the blood outflow cage each have a first radius between 0.07 mm and 0.21 mm, and the outer edges of the struts of the blood outflow cage each have a second radius between 0.07 mm and 0.21 mm.
10. The blood outflow cage of para 1, wherein the blood outflow cage is manufactured from a single piece of metal or polymer.
11. A blood pump assembly sized for passage through a vascular lumen of a patient, comprising:
   a pump;
   an impeller blade rotatably coupled to the pump;
   a blood inflow cage extending about a rotation axis of the impeller blade, and having at least two blood inlet openings;
   a cannula in fluid communication with the first housing, the proximal end of the cannula being coupled to the blood inflow cage; and
   a blood outflow cage coupled to the distal end of the cannula, including:
      a distal cap with a substantially conical, substantially curved-conical, or substantially dome-shaped outer surface;
      a diffuser having a substantially conical or substantially curved-conical outer surface, wherein the diffuser is disposed proximal to the distal cap; and
      a peripheral wall having:
         an inner surface;
         an outer surface;
         two or more blood exhaust openings, each blood exhaust opening being defined by two struts, wherein each strut has rounded inner and outer edges and rounded junctions with the diffuser, and wherein the proximal end of each blood exhaust opening has rounded corners; and
            a first cylindrical section that abuts the proximal end of each strut.
12. The blood pump assembly of para 11, further comprising an aperture through the diffuser and the distal cap of the blood outflow cage.
13. The blood pump assembly of para 11, wherein the peripheral wall of the blood outflow cage tapers in thickness from a first thickness at the struts to a second thickness at the first cylindrical section, wherein the first thickness is greater than the second thickness.
14. The blood pump assembly of para 11, wherein the peripheral wall of the blood outflow cage further comprises a second cylindrical section that abuts the first cylindrical section, and wherein an outer diameter of the second cylindrical section is smaller than an outer diameter of the first cylindrical section.
15. The blood pump assembly of para 11, further comprising a flexible tubular extension coupled to the distal cap of the blood outflow cage.
16. The blood pump assembly of para 15, wherein the distal end of the flexible tubular extension is curved in its relaxed state.
17. The blood pump assembly of para 11, further including a fillet between an inner surface of each strut and the diffuser of the blood outflow cage.
18. The blood pump assembly of para 17, wherein the fillet has a radius of approximately 0.30 mm.
19. The blood pump assembly of para 11, wherein the inner edges of the struts of the blood outflow cage each have a first radius between 0.07 mm and 0.21 mm, and the outer edges of the struts of the blood outflow cage each have a second radius between 0.07 mm and 0.21 mm.
20. The blood pump assembly of para 1, wherein the blood outflow cage is manufactured from a single piece of metal or polymer.

## Claims

1. A blood outflow cage for an intravascular blood pump assembly and sized for passage through a vascular lumen of a patient, comprising:
a distal cap with a substantially conical, substantially curved-conical, or substantially dome-shaped outer surface;
a diffuser having a substantially conical or substantially curved-conical outer surface, wherein the diffuser is disposed proximal to the distal cap; and
a peripheral wall having:
an inner surface;
an outer surface;
two or more blood exhaust openings, each blood exhaust opening being defined by two struts, wherein each strut has rounded inner and outer edges and rounded junctions with the diffuser, and wherein the proximal end of each blood exhaust opening has rounded corners; and
a first cylindrical section that abuts the proximal end of each strut.

2. The blood outflow cage of claim 1, further comprising an aperture through the diffuser and the distal cap.

3. The blood outflow cage of claim 1, wherein the peripheral wall tapers in thickness from a first thickness at the struts to a second thickness at the first cylindrical section, wherein the first thickness is greater than the second thickness.

4. The blood outflow cage of claim 1, wherein the peripheral wall further comprises a second cylindrical section that abuts the first cylindrical section, and wherein an outer diameter of the second cylindrical section is smaller than an outer diameter of the first cylindrical section.

5. The blood outflow cage of claim 1, further comprising a flexible tubular extension coupled to the distal cap, wherein preferably the distal end of the flexible tubular extension is curved in its relaxed state.

6. The blood outflow cage of claim 1, further comprising a fillet between an inner surface of each strut and the diffuser, wherein preferably the fillet has a radius of approximately 0.30 mm.

7. The blood outflow cage of claim 1, wherein the inner edges of the struts of the blood outflow cage each have a first radius between 0.07 mm and 0.21 mm, and the outer edges of the struts of the blood outflow cage each have a second radius between 0.07 mm and 0.21 mm.

8. The blood outflow cage of claim 1, wherein the blood outflow cage is manufactured from a single piece of metal or polymer.

9. A blood pump assembly sized for passage through a vascular lumen of a patient, comprising:
a pump;
an impeller blade rotatably coupled to the pump;
a blood inflow cage extending about a rotation axis of the impeller blade, and having at least two blood inlet openings;
a cannula in fluid communication with the first housing, the proximal end of the cannula being coupled to the blood inflow cage; and
a blood outflow cage coupled to the distal end of the cannula, including:
a distal cap with a substantially conical, substantially curved-conical, or substantially dome-shaped outer surface;
a diffuser having a substantially conical or substantially curved-conical outer surface, wherein the diffuser is disposed proximal to the distal cap; and
a peripheral wall having:
an inner surface;
an outer surface;
two or more blood exhaust openings, each blood exhaust opening being defined by two struts, wherein each strut has rounded inner and outer edges and rounded junctions with the diffuser, and wherein the proximal end of each blood exhaust opening has rounded corners; and
a first cylindrical section that abuts the proximal end of each strut.

10. The blood pump assembly of claim 9, further comprising an aperture through the diffuser and the distal cap of the blood outflow cage.

11. The blood pump assembly of claim 9, wherein the peripheral wall of the blood outflow cage tapers in thickness from a first thickness at the struts to a second thickness at the first cylindrical section, wherein the first thickness is greater than the second thickness.

12. The blood pump assembly of claim 9, wherein the peripheral wall of the blood outflow cage further comprises a second cylindrical section that abuts the first cylindrical section, and wherein an outer diameter of the second cylindrical section is smaller than an outer diameter of the first cylindrical section.

13. The blood pump assembly of claim 9, further comprising a flexible tubular extension coupled to the distal cap of the blood outflow cage, wherein preferably the distal end of the flexible tubular extension is curved in its relaxed state.

14. The blood pump assembly of claim 9, further including a fillet between an inner surface of each strut and the diffuser of the blood outflow cage, wherein preferably the fillet has a radius of approximately 0.30 mm.

15. The blood pump assembly of claim 9, wherein the inner edges of the struts of the blood outflow cage each have a first radius between 0.07 mm and 0.21 mm, and the outer edges of the struts of the blood outflow cage each have a second radius between 0.07 mm and 0.21 mm, or wherein the blood outflow cage is manufactured from a single piece of metal or polymer.
